# FASCICULE DE BREVET EUROPEEN

(11) **EP 4 052 748 B1**
(45) Date de publication et mention de la délivrance du brevet: **18.06.2025**
(21) Numéro de dépôt: 22155326.6
(22) Date de dépôt: 07.02.2022
(51) Int. Cl.: A61M 16/00, A61M 16/08, A61M 16/20

(54) **INTERFACE DE VENTILATION DE PATIENT DESTINÉE À ÊTRE COUPLÉE À UN VENTILATEUR MÉDICAL ET À UNE SOURCE DE GAZ**
SCHNITTSTELLE FÜR DIE PATIENTENBEATMUNG ZUR KOPPLUNG AN EIN MEDIZINISCHES BEATMUNGSGERÄT UND EINE GASQUELLE
INTERFACE FOR VENTILATING A PATIENT INTENDED TO BE COUPLED TO A MEDICAL VENTILATOR AND A GAS SOURCE

(30) Priorité: 04.03.2021 FR 2102111
(43) Date de publication de la demande: 07.09.2022
(73) Titulaire: L'Air Liquide, société anonyme pour l'Étude et l'Exploitation des procédés Georges Claude, 75007 Paris (FR)
(72) Inventeur: BOULANGER, Thierry, Newark, 19702 (US)
(74) Mandataire: Air Liquide

(56) Documents cités:
- WO-A1-99/10034
- DE-A1- 2 035 829
- FR-A- 1 487 768
- US-A- 5 651 360
- US-A- 5 769 072

## Description

La présente invention concerne un dispositif ou interface de ventilation destiné à être couplé à un ventilateur médical, c'est-à-dire un appareil d'assistance ventilatoire, de manière à assurer une délivrance de différents mélanges gazeux à un être humain, typiquement un patient.

Les gaz médicaux à un ou plusieurs constituants (i.e. gaz pur ou mélange gazeux) sont couramment utilisés par inhalation pour traiter différentes conditions physiologiques ou pathologies respiratoires chez les patients de tout âge, i.e. adulte, adolescent, enfant ou nourrisson.

Ainsi, l'air enrichi en oxygène est utilisé pour traiter les situations hypoxémiques, les mélanges hélium/O₂ sont utilisés pour traiter les exacerbations de BPCO ou les crises d'asthme aigüe, et les mélanges protoxyde d'azote/O₂ (i.e. N₂O/O₂) sont utilisés pour traiter la douleur et l'anxiété, par exemple lors de soins dentaires ou d'opérations légères aux urgences.

Chez les patients plus « critiques », on utilise des ventilateurs médicaux, aussi appelés appareils d'assistance respiratoire ou de ventilation assistée ou analogue, spécialement adaptés aux soins critiques. Ainsi, le xénon (Xe) est parfois utilisé en tant anesthésique gazeux, pendant une opération chirurgicale de personnes fragiles. De même, l'argon ou l'hydrogène sont parfois utilisés dans des indications spécifiques.

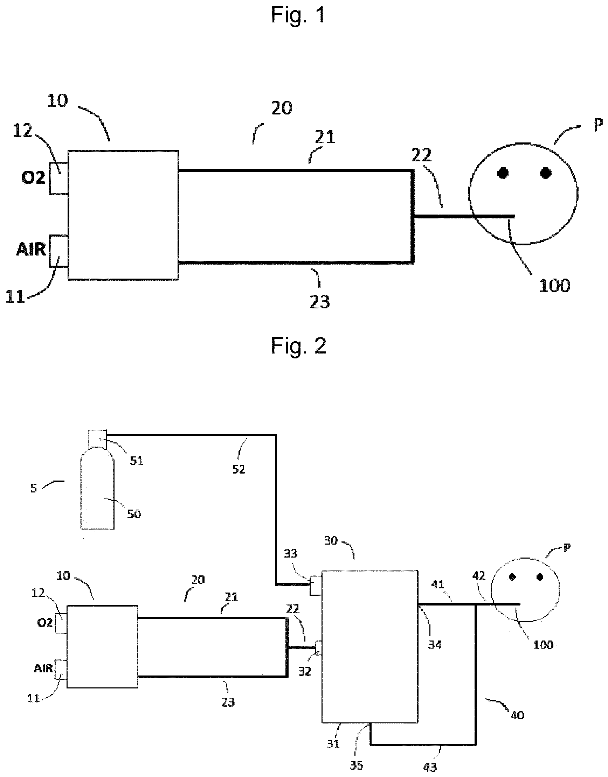

Or, comme expliqué ci-après en rapport avec Fig. 1, les ventilateurs médicaux existants ne sont souvent pas adaptés et/ou conçus pour permettre d'administrer plusieurs types de gaz thérapeutiques différents, en particulier les mélanges gazeux. Ainsi, un ventilateur médical dédié à la fourniture d'air ou d'air enrichi en O₂ ne sera en général pas utilisable pour délivrer un autre gaz médical, par exemple un mélange Ar/O₂, N₂O/O₂ ou autres, du fait des particularités chimiques propres à ces différents gaz, comme leur viscosité, leur conductivité thermique ou leur chaleur massique.

Ceci oblige les hôpitaux à acquérir différents types de ventilateurs médicaux afin de pouvoir assurer la fourniture de mélanges gazeux différents à leurs patients, en fonction de leurs pathologies ou conditions médicales, à défaut de quoi le traitement de certains patients ne peut être assuré.

Ceci n'est évidemment pas satisfaisant.

FR1487768 enseigne un appareil de respiration artificielle comprenant une première ligne d'acheminement de gaz avec une première chambre inspiratoire contenant une vessie inspiratoire à volume interne variable en communication fluidique avec la ligne d'acheminement de gaz, et une seconde ligne d'acheminement de gaz en communication fluidique avec la première chambre inspiratoire et une chambre expiratoire contenant une vessie expiratoire à volume interne variable. Une ligne expiratoire est en communication fluidique avec le volume interne variable de la vessie expiratoire.

Ce type d'appareil engendre des problèmes de sécurité ventilatoire pour le patient. En effet, il est nécessaire de remplir la vessie inspiratoire pendant l'expiration du patient, ce qui laisse place au risque que la vessie ne puisse avoir le temps de se remplir entièrement, par exemple en cas d'asynchronie entre le patient et l'appareil, ou lorsque plusieurs cycles inspiratoires se succèdent rapidement (phénomène appelé auto-déclenchement ou *« auto-triggering »* en anglais). Dès lors, le patient pourrait être exposé à une vessie inspiratoire complètement vide, donc ne pouvant fournir de gaz au patient, ce qui n'est pas acceptable. De plus, cet appareil n'est pas adapté à une administration de plusieurs types de gaz thérapeutiques différents avec un même appareil.

Le problème est donc de pouvoir administrer plusieurs types de gaz thérapeutiques différents avec un même ventilateur médical, même lorsque ce ventilateur médical n'a pas été conçu ou n'est à priori pas adapté à une administration de ces gaz thérapeutiques différents, de manière à pouvoir utiliser un même ventilateur médical pour fournir du gaz à des patients souffrant de différentes pathologies respiratoires et/ou ayant besoin d'inhaler des gaz thérapeutiques de compositions différentes, en particulier des gaz médicaux choisis parmi l'air, l'oxygène ou les mélanges air/O₂, Ar/O₂, Xe/O₂, N₂O/O₂ ou autres. Avantageusement, l'administration de gaz doit se faire sans engendrer de risque pour le patient, en particulier sans risque de ne pas fournir de gaz respiratoire au patient.

Une solution de l'invention concerne alors une interface de ventilation comprenant au moins :
- une ligne d'acheminement de gaz principal comprenant un premier port d'entrée de gaz pour recevoir du gaz fourni par un ventilateur médical ;
- une ligne d'acheminement de gaz additionnel comprenant un second port d'entrée de gaz pour recevoir du gaz fourni par une source de gaz additionnelle ; et
- une ligne expiratoire ;
- la ligne d'acheminement de gaz additionnel comprenant une première chambre inspiratoire et une seconde chambre inspiratoire agencées en parallèle l'une de l'autre ;
   - la première chambre inspiratoire contenant un premier réservoir inspiratoire à volume interne variable agencé au sein d'une première enceinte inspiratoire, le volume interne variable du premier réservoir inspiratoire étant en communication fluidique avec la ligne d'acheminement de gaz additionnel, et
   - la seconde chambre inspiratoire contenant un second réservoir inspiratoire à volume interne variable agencé au sein d'une seconde enceinte inspiratoire, le volume interne variable du second réservoir inspiratoire étant en communication fluidique avec la ligne d'acheminement de gaz additionnel,
- la ligne expiratoire comprenant une première chambre expiratoire et une seconde chambre expiratoire agencées en parallèle l'une de l'autre ;
   - la première chambre expiratoire contenant un premier réservoir expiratoire à volume interne variable agencé au sein d'une première enceinte expiratoire, le volume interne variable du premier réservoir expiratoire étant en communication fluidique avec la ligne expiratoire ; et
   - la seconde chambre expiratoire comprenant un second réservoir expiratoire à volume interne variable agencé au sein d'une seconde enceinte expiratoire, le volume interne variable du second réservoir expiratoire étant en communication fluidique avec la ligne expiratoire ;
- et la ligne d'acheminement de gaz principal étant en communication fluidique avec les volumes internes des premières et secondes enceintes inspiratoires et expiratoires.

Selon le mode de réalisation considéré, l'interface de l'invention peut comprendre l'une ou plusieurs des caractéristiques suivantes :
- la ligne d'acheminement de gaz additionnel et/ou la ligne d'acheminement de gaz principal comprennent un ou des passages de gaz, conduits de gaz ou analogues.
- les premières et secondes enceintes inspiratoires et expiratoires sont hermétiques (i.e. fluidiquement étanches) et rigides, de préférence de forme générale cylindrique.
- le premier et/ou le second réservoir inspiratoire et/ou le premier et/ou le second réservoir expiratoire comprennent des soufflets, de préférence circulaires.
- le premier et/ou le second réservoir inspiratoire et/ou le premier et/ou le second réservoir expiratoire sont formés d'au moins un matériau souple, de préférence de silicone de grade médical.
- la ligne d'acheminement de gaz additionnel se ramifie en un premier tronçon de ligne et un second tronçon de ligne, le premier tronçon de ligne comprenant la première chambre inspiratoire et le second tronçon de ligne comprenant la seconde chambre inspiratoire.
- le premier tronçon de ligne comprend une première vanne d'admission de gaz et le second tronçon de ligne comprend une seconde vanne d'admission de gaz.
- la ligne expiratoire se ramifie en un premier canal de collecte et un second canal de collecte, le premier canal de collecte comprenant la première chambre expiratoire et le second canal de collecte comprenant la seconde chambre expiratoire.
- la première chambre inspiratoire comprend un premier capteur inspiratoire.
- la seconde chambre inspiratoire comprend un second capteur inspiratoire.
- la première chambre expiratoire comprend un premier capteur expiratoire.
- la seconde chambre expiratoire comprend un second capteur expiratoire.
- lesdits premier et second capteurs inspiratoires et expiratoires sont reliés électriquement à des moyens de pilotage.
- les moyens de pilotage pilotent au moins les première et seconde vannes d'admission de gaz.
- elle comprend un boitier externe rigide, aussi appelé enveloppe ou carcasse, par exemple en polymère.
- les moyens de pilotage comprennent au moins une carte de commande électronique et/ou au moins une unité de contrôle à microprocesseur, typiquement un microcontrôleur.
- elle comprend des moyens d'alimentation électrique, telle qu'une batterie ou un raccordement électrique au secteur.
- les moyens d'alimentation électrique alimentent les moyens de pilotage en courant électrique.
- les moyens de pilotage pilotent une ou plusieurs vannes ou sélecteurs, en particulier des vannes tout ou rien 3:2, ou des vannes 4:2.

L'invention concerne en outre une installation de ventilation assistée d'un patient comprenant :
- une interface de ventilation selon l'invention,
- un ventilateur médical relié fluidiquement au premier port d'entrée de gaz de la ligne d'acheminement de gaz principal, via un premier circuit patient, et
- une source de gaz additionnelle reliée fluidiquement au second port d'entrée de gaz de la ligne d'acheminement de gaz additionnel, via un second circuit patient.

Selon le mode de réalisation considéré, l'installation de ventilation assistée selon l'invention peut comprendre l'une ou plusieurs des caractéristiques suivantes :
- la source de gaz additionnelle contient un gaz ou mélange gazeux à base d'argon, en particulier un mélange argon/O₂, ou à base de xénon, en particulier un mélange xénon/azote/O₂.
- la source de gaz additionnelle est une bouteille de gaz sous pression.
- le premier circuit patient et/ou le second circuit patient comprennent des conduites de gaz ou analogues.
- elle comprend une interface respiratoire patient, tel un masque respiratoire, une sonde trachéale ou autre, en communication fluidique avec le second circuit patient.

L'invention va maintenant être mieux comprise grâce à la description détaillée suivante, faite à titre illustratif mais non limitatif, en référence aux figures annexées parmi lesquelles :
Fig. 1 schématise un ventilateur de soins critiques classique fournissant du gaz à un patient, via un circuit patient.
Fig. 2 schématise l'insertion d'un mode de réalisation d'une interface ventilatoire selon l'invention entre le ventilateur et le patient de Fig. 1.
Fig. 3 schématise une architecture de l'interface ventilatoire selon l'invention de Fig. 2.
Fig. 4 à Fig. 8 illustrent le fonctionnement de l'interface ventilatoire selon l'invention illustrée en Fig. 2 et Fig. 3.
Fig. 9 illustre les principaux éléments l'interface ventilatoire illustrée en Fig. 2 et Fig. 3, en configuration initiale.
Fig. 10 à Fig. 13 illustrent le fonctionnement l'interface ventilatoire illustrée en Fig. 2 et Fig. 3 couplée à un ventilateur médical et à une source de gaz additionnel.

Fig. 1 schématise un appareil d'assistance respiratoire ou ventilateur médical 10, tel un ventilateur de soins critiques, classique fournissant du gaz à un patient P.

Ce ventilateur médical 10 est relié fluidiquement au patient P par l'intermédiaire d'un circuit patient 20 comprenant une branche inspiratoire 21 et une branche expiratoire 23, se rejoignant au niveau d'un embranchement 22, telle une pièce en Y, prolongé par une interface respiratoire patient 100, tel qu'une sonde d'intubation, un masque naso-buccal ou facial, ou autre...

Un tel ventilateur médical 10 est connecté via des entrées de gaz 11, 12, à deux sources de gaz de qualité médicale, par exemple de l'air (reliée en 11) et de l'oxygène (reliée en 12), provenant classiquement du réseau hospitalier.

Le ventilateur 10 est muni d'organes de commande (non représentés) tels qu'une interface tactile et/ou des boutons permettant à un utilisateur, typiquement un médecin ou le personnel infirmier, de sélectionner et régler les paramètres de ventilation du patient P. Ces paramètres peuvent comprendre la fraction inhalée en oxygène (FiO₂), définissant le rapport d'apport gazeux entre la source d'air et la source d'O₂, la fréquence respiratoire, c'est-à-dire le rythme auquel le ventilateur va fournir le gaz au patient afin d'assurer, tout ou partiellement, les phases inspiratoires du patient P considéré. Cette phase inspiratoire peut être matérialisée sous forme d'une pression positive ou bien d'un volume pouvant être également réglés par l'utilisateur. D'autres paramètres comme la pression expiratoire positive, le temps de la phase inspiratoire etc... font aussi partie des paramètres usuellement réglés. Cet ensemble de réglages détermine les paramètres de ventilation du patient par le ventilateur 10.

Pendant chaque phase inspiratoire du patient, le ventilateur 10 délivre dans la branche inspiratoire 21 du premier circuit patient 20, le volume ainsi réglé. Afin qu'une portion de ce volume ne s'échappe par la branche expiratoire 23 du circuit patient 20, le ventilateur actionne des mécanismes internes permettant de condamner toute circulation de gaz dans la branche expiratoire 23. La totalité du volume gazeux est alors dirigée dans l'embranchement 22 et, in fine, inhalé par le patient P via l'interface respiratoire 100.

Ensuite, lors de chaque phase expiratoire, le ventilateur 10 ouvre la branche expiratoire 23 dans laquelle le patient P est libre d'expirer et condamne, dans le même temps, la branche inspiratoire 21 afin d'éviter que les gaz expirés n'y circulent en sens inverse. Le ventilateur 10 mesure en outre la quantité de gaz expiré pour s'assurer que le volume restitué est similaire au volume inspiré et qu'ainsi il n'y a pas de fuites au niveau de l'interface respiratoire 100, c'est-à-dire de défauts d'étanchéité gazeuse.

Ce processus se répète de façon cyclique selon la fréquence respiratoire du patient P, c'est-à-dire l'alternance de ses phases inspiratoires et expiratoires.

En théorie, l'entrée de gaz 11 du ventilateur pourrait être reliée à une autre source de gaz additionnel (i.e. autre que de l'air), alimentant le ventilateur 1, par exemple un mélange binaire de type argon/O₂ ou ternaire de type xénon/azote/O₂.

Cependant, en pratique, une telle substitution est problématique car les propriétés physiques de ces mélanges (i.e. densité, conductivité thermique...) sont différentes de celle de l'air pour lequel le ventilateur 10 a été conçu et validé, et dès lors, alimenter l'entrée 12 avec un mélange gazeux autre que de l'air, peut engendrer des erreurs plus ou moins significatives au cours de la délivrance du gaz au patient (i.e. erreurs de volumes inspirés ou de concentration d'O₂...) et par ailleurs déclencher des alarmes intempestives au niveau du ventilateur 10.

Afin de résoudre ces problèmes, selon l'invention, une interface de ventilation 30 faisant office de système d'interfaçage est raccordée, d'une part, au ventilateur 10 et à une source de gaz additionnel 5, i.e. un gaz autre que l'air, telle une bouteille de gaz sous pression contenant un mélange argon/O₂ ou xénon/azote/O₂, et d'autre part, au patient P, comme expliqué ci-après et illustré sur Fig. 2 à Fig. 9.

Plus précisément, comme schématisé sur Fig. 2, l'interface de ventilation 30 est un dispositif faisant office de système d'interfaçage. Elle comprend un boitier ou enveloppe externe rigide 31, par exemple en polymère, portant un premier port d'entrée ou de raccordement 32 configuré pour permettre un raccordement fluidique à la première pièce en Y 22 du premier circuit patient 20 qui est lui-même connecté fluidiquement à un ventilateur médical 10, tel celui de Fig. 1, comme expliqué ci-avant, et par ailleurs un second port d'entrée ou de raccordement 33 configuré pour permettre un raccordement fluidique à la source de gaz additionnel 5.

Par ailleurs, l'interface de ventilation 30 comprend en outre des premier et second ports de raccordement 34, 35, agencés par exemple sur le boitier 31, lesquels servent au raccordement fluidique d'un second circuit patient 40, comme expliqué ci-après.

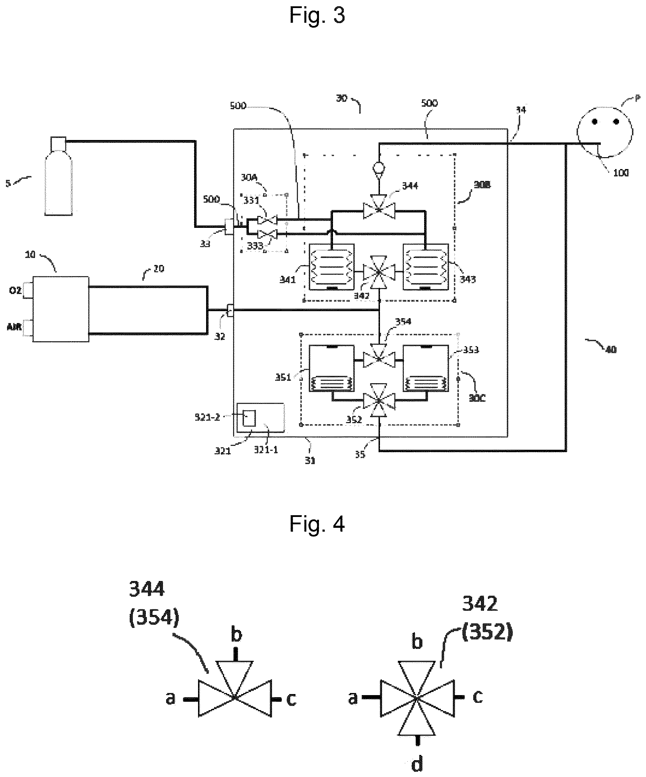

Comme on le voit sur Fig. 3, au moins une ligne d'acheminement de gaz additionnel 500 relie fluidiquement le second port d'entrée de gaz 33 au premier port de raccordement 34. Cette ligne d'acheminement de gaz additionnel 500 comprend plusieurs tronçons de ligne 330, 332, 334, 411, 3413, 3433, y compris une ramification en deux tronçons de lignes parallèles 332, 334, à savoir un premier tronçon de ligne 330 et un second tronçons de ligne 332, comme expliqué ci-après.

La source de gaz 5 est, par exemple, une bouteille de gaz 50 contenant un mélange gazeux thérapeutique qui y est stocké sous pression, de préférence jusqu'à 200 ou 300 bar abs, tel un mélange gazeux argon/O₂ ou xénon/azote/O₂. Elle est équipée d'un robinet à détendeur intégré 51 (RDI) qui permet de détendre le gaz à une pression inférieure d'utilisation, par exemple de l'ordre de 4 à 5 bar abs. La bouteille 50 est reliée fluidiquement, via un flexible de raccordement 52, au second port d'entrée de gaz 33 de l'interface de ventilation 30.

Par ailleurs, un second circuit patient 40 est raccordé à l'interface de ventilation 30. Il comprend une seconde branche inspiratoire 41, une seconde branche expiratoire 43 ainsi qu'une seconde pièce Y 42 prolongée par l'interface respiratoire 100 placée au niveau du patient P, comme illustré en Fig. 2. Les secondes branches inspiratoire 41 et expiratoire 43 vient se raccorder fluidiquement aux premier et second ports de raccordement 34, 35 de l'interface de ventilation 30.

Un mode de réalisation de l'architecture interne de l'interface de ventilation 30 selon l'invention, est schématisée en Fig. 3.

L'interface de ventilation 30 comprend des moyens de pilotage 321 comprenant une carte de commande électronique 321-1 et une unité de contrôle à microprocesseur 321-2, typiquement un microcontrôleur.

Tous les éléments électromécaniques de l'interface de ventilation 30 sont alimentés électriquement et commandés par les moyens de pilotage 321, lesquels sont eux-mêmes alimentés électriquement par une source de courant électrique (non montrée), par exemple une liaison au courant du secteur de type cordon électrique et prise de raccordement, ou une (ou des) batterie d'alimentation électrique, de préférence rechargeable.

La carte de commande 321-1 intègre préférentiellement l'unité de contrôle 321-2 et est configurée pour commander et par ailleurs analyser les signaux provenant des différents composants de l'interface de ventilation 30, tels que vannes, capteurs...

Par ailleurs, l'interface de ventilation 30 comprend trois modules principaux agencés dans l'enveloppe 31, à savoir :
- un module d'admission du gaz thérapeutique 30A, comprenant une première et une deuxième vanne d'admission 331, 333 de gaz thérapeutique,
- un module de délivrance du gaz thérapeutique, appelé aussi « module inspiratoire » 30B, comprenant une première et une seconde chambre inspiratoire 341, 343, un premier sélecteur 342 et une première vanne 344, et
- un module de collecte des gaz expirés, appelé aussi module « expiratoire » 30C, comprenant une première et une deuxième chambre expiratoire 351, 353, un deuxième sélecteur 352 et une deuxième vanne 354.

La première vanne d'admission 331 est une vanne tout ou rien normalement fermée (i.e. en position de repos) qui s'ouvre en réponse à une commande par les moyens de pilotage 321 et permet, selon son état, d'injecter du gaz provenant de la source de gaz thérapeutique 5.

Fig. 4 décrit le fonctionnement des premier et second sélecteurs 342, 352 ainsi que des première et seconde vannes 344, 354.

En position de repos, la première vanne 344 (et similairement 354) réalise une connexion fluidique entre le port « a » et le port « b », occluant le port « c ». En réponse à une commande des moyens de pilotage 321, la première vanne 344 (et similairement 354) bascule en réalisant une communication fluidique entre le port « b » et le port « c », occluant alors le port « a ». De la même manière, en position de repos, le premier sélecteur 342 (et similairement 352) réalise une double communication fluidique entre d'une part, les ports « a » et « d » et, d'autre part, les ports « b » et « c ».

En réponse à une commande des moyens de pilotage 321, le premier sélecteur 342 (et similairement 352) bascule en réalisant une double communication fluidique entre d'une part les ports « a » et « b » et, d'autre part, les ports « c » et « d ».

Les première et seconde vannes 344, 354 sont préférentiellement des vannes tout ou rien 3:2, et les premier et second sélecteurs 342, 352 sont préférentiellement des vannes 4 :2. De telles vannes sont disponibles auprès de la société IMI NORGREN^{®} par exemple.

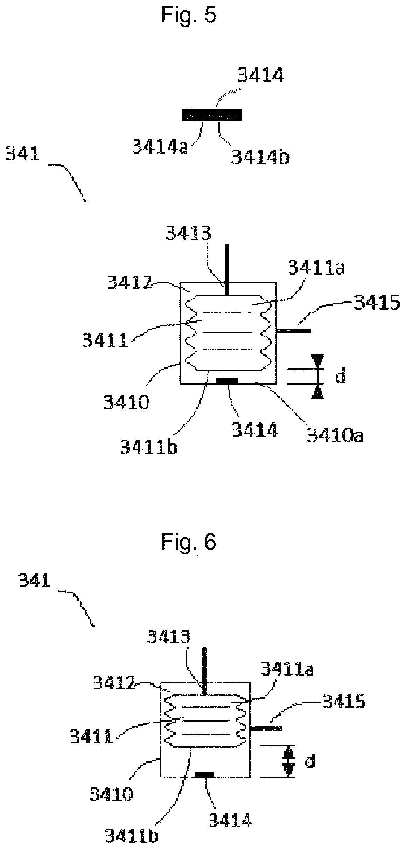

La première chambre inspiratoire 341 est décrite en Fig. 5. Elle comprend une première enveloppe ou enceinte inspiratoire 3410, hermétique et rigide, et de préférence cylindrique dans laquelle est agencé un premier réservoir de gaz inspiratoire 3411 déformable, se présentant préférentiellement sous forme de soufflets circulaires ou analogues.

Le premier réservoir de gaz inspiratoire 3411 utilise un matériau souple, par exemple un matériau de type silicone de grade médical, ayant une épaisseur comprise entre 0.25 et 0.5 mm par exemple.

Au repos, le premier réservoir de gaz inspiratoire 3411 peut renfermer un volume de gaz V de l'ordre de 1L par exemple. Il présente un premier canal d'évacuation 3413. Il est fluidiquement isolé de la première enveloppe ou enceinte inspiratoire 3410, ou plus particulièrement isolé du premier volume interne inspiratoire 3412 de la première enveloppe ou enceinte inspiratoire 3410 entourant ledit premier réservoir de gaz inspiratoire 3411.

Par ailleurs, la première enveloppe ou enceinte inspiratoire 3410 présente une première entrée de gaz 3415, fluidiquement connectée au premier volume interne inspiratoire 3412 de ladite première inspiratoire 3410.

Par ailleurs, un premier capteur inspiratoire 3414, préférentiellement un capteur de distance inspiratoire, est agencé à proximité du premier réservoir de gaz inspiratoire 3411 de manière à coopérer avec ce dernier et pouvoir mesurer le degré de gonflage, i.e. gonflage/dégonflage, du premier réservoir de gaz inspiratoire 3411 à volume variable. Le premier capteur 3414 est agencé dans la première enveloppe ou enceinte inspiratoire 3410 de la première chambre inspiratoire 341 de l'interface respiratoire 3, par exemple fixé sur la paroi interne 3410a de la première enveloppe ou enceinte inspiratoire 3410.

Dans le mode de réalisation proposé, le premier capteur 3414 est de préférence un capteur de temps de vol *ou « time of flight* sensor » (en anglais) comprenant un émetteur de signal 3414a, de préférence de signal lumineux, telle une diode laser, et un récepteur de signal 3414b, de préférence de signal lumineux, telle une photodiode.

A intervalles réguliers, par exemple toutes les 50 ms, l'émetteur 3414a génère un signal, tel un signal lumineux, aussi appelé 'pulsation' ou 'pulse', en direction du premier réservoir de gaz inspiratoire 3411, pour atteindre une région donnée de paroi 3411b située sur la surface périphérique de la paroi externe 3411a du premier réservoir de gaz inspiratoire 3411. Une fraction au moins du signal lumineux émis est réfléchi par la région de paroi 3411b du premier réservoir de gaz inspiratoire 3411 et frappe en retour le récepteur 3414b. Le « temps de vol », c'est-à-dire la durée aller/retour, séparant l'émission de la réception du signal est proportionnelle à une distance (d) variable entre le premier capteur de distance inspiratoire 3414 et la région de paroi 3411b.

Plus ce temps est faible, plus proche du premier capteur de distance inspiratoire 3414 est la région de paroi 3411b, donc plus le premier réservoir de gaz inspiratoire 3411 est rempli de gaz, i.e. gonflé et sa paroi périphérique tendue, et inversement, plus le temps est important, plus éloignée du premier capteur de distance inspiratoire 3414 est la région de paroi 3411b, donc moins le premier réservoir de gaz inspiratoire 3411 contient de gaz, i.e. plus il est dégonflé et sa paroi périphérique distendue.

En d'autres termes, le premier capteur de distance inspiratoire 3414 permet d'apprécier le niveau ou degré de gonflage du premier réservoir de gaz inspiratoire 3411, donc d'évaluer le volume V de gaz contenu dans le premier réservoir de gaz inspiratoire 3411.

Fig. 5 à Fig. 7 illustrent ainsi différents degrés de gonflage, i.e. de gonflage/dégonflage, du premier réservoir de gaz inspiratoire 3411, et la coopération établie entre le premier capteur de distance inspiratoire 3414 et le premier réservoir de gaz inspiratoire 3411.

Comme on peut le voir, la distance d varie, i.e. n'est pas toujours la même, en fonction de la quantité de gaz qui se trouve dans le premier réservoir de gaz inspiratoire 3411, c'est-à-dire en fonction du degré de gonflage du premier réservoir de gaz inspiratoire 3411.

Fig. 5 montre le premier réservoir de gaz inspiratoire 3411 déformable, i.e. à volume variable, dans son état initial, c'est-à-dire au repos, pendant lequel le premier capteur de distance inspiratoire 3414 effectue une (des) mesure par émission/réception d'un signal lumineux. Comme expliqué auparavant, le temps de vol ou de transit aller-retour du pulse lumineux détermine la distance d séparant le premier capteur de distance inspiratoire 3414 de la région de paroi 3411b du premier réservoir de gaz inspiratoire 3411. Cette distance, appelée D_{REPOS} est minimale, et correspond à un volume de gaz maximal V_{MAX} dans le premier réservoir de gaz inspiratoire 3411.

Fig. 6 illustre un début de vidange du premier réservoir de gaz inspiratoire 3411, par exemple lorsque la pression dans le premier volume interne inspiratoire 3412 augmente, du par exemple à l'apport de gaz via la première entrée de gaz 3415. En réponse à cet apport de gaz dans le premier volume interne inspiratoire 3412, le premier réservoir va se déformer et plus particulièrement réduire son volume interne V en expulsant une portion de ce dit volume interne dans le premier canal d'évacuation 3413. Cette réduction de volume persiste jusqu'à ce que les pressions régnant dans le premier volume interne inspiratoire 3412 et le premier réservoir de gaz inspiratoire 3411 (et donc le premier canal d'évacuation 3413) s'équilibrent. Pour être plus explicite, si l'apport de gaz par la première entrée de gaz 3415 est un volume V1, alors le même volume V1 sera évacué du premier réservoir de gaz inspiratoire 3411 par le premier canal d'évacuation 3413. Dans la configuration illustrée en Fig. 6, le premier capteur de distance inspiratoire 3414 effectue à nouveau une (des) mesure de laquelle il ressort que la distance d mesurée est/devient supérieure à la distance D_{REPOS}. Elle correspond, par exemple, à un volume V de gaz dans le premier réservoir de gaz inspiratoire 3411 réduit de moitié par rapport au volume maximal V_{MAX}, par exemple de l'ordre de 500 ml.

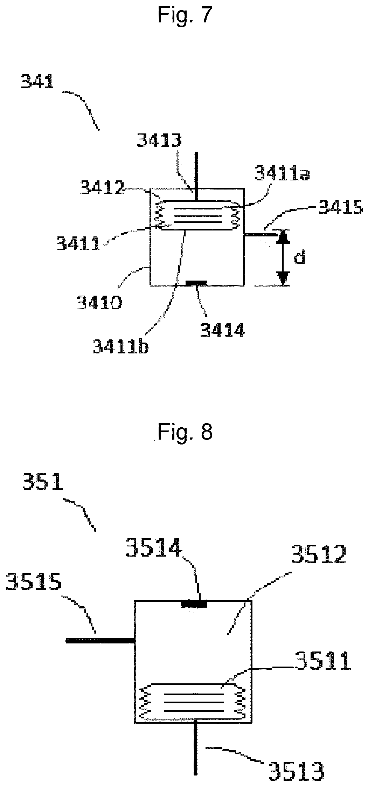

Fig. 7 représente le premier réservoir de gaz inspiratoire 3411 totalement dégonflé, c'est-à-dire ne contenant plus de gaz. Là encore, le premier capteur de distance inspiratoire 3414 effectue une (des) mesure de distance comme précédemment. Il en ressort alors une valeur de distance d mesurée maximale.

On comprend donc, au vu des Fig. 5 à Fig. 7, que la distance d est proportionnelle au gonflage du premier réservoir de gaz inspiratoire 3411, c'est-à-dire que plus le premier réservoir de gaz inspiratoire 3411 contient une quantité (i.e. volume) de gaz importante, plus la distance d mesurée est faible. Il est donc possible de corréler la mesure de distance d issue du premier capteur de distance inspiratoire 3414 au volume de gaz V contenu dans le premier réservoir de gaz inspiratoire 3411, i.e. à son taux ou degré de remplissage.

Enfin, Fig. 8 représente la première chambre expiratoire 351, laquelle est identique à la première chambre inspiratoire 341, à l'exception près d'avoir subi une rotation par rapport à un axe de symétrie horizontal. Ainsi, la première chambre expiratoire 351 comprend également une première enveloppe ou enceinte expiratoire 3510 dans laquelle est agencé un premier réservoir de gaz expiratoire 3511. A l'inverse du premier réservoir de gaz inspiratoire 3411, ce premier réservoir de gaz expiratoire 3511 renferme un volume de gaz nul au repos, c'est-à-dire le premier réservoir de gaz expiratoire 3511 s'effondre sous son propre poids dû à l'effet de la gravité. Le premier réservoir de gaz expiratoire 3511 présente un premier canal de collecte 3513, en ce sens que ledit premier réservoir de gaz expiratoire 3511 est également fluidiquement isolé de la première enveloppe ou enceinte expiratoire 3510, c'est-à-dire isolé du premier volume interne expiratoire 3512 de la première enveloppe ou enceinte expiratoire 3510, entourant ledit premier réservoir de gaz expiratoire 3511. Par ailleurs, la première enveloppe ou enceinte expiratoire 3510 présente une première sortie de gaz 3515, fluidiquement connectée au premier volume interne expiratoire 3512 de ladite première enveloppe ou enceinte expiratoire 3510.

Comme précédemment, un premier capteur expiratoire 3514, tel un capteur de distance, est agencé dans la première enveloppe ou enceinte expiratoire 3510 afin de mesurer la distance « d » le séparant du premier réservoir de gaz expiratoire 3511, comme expliqué ci-avant en références aux Fig.5 à Fig. 7. Toutefois, contrairement à la première chambre inspiratoire 341, la mesure de distance retournée par le premier capteur de distance expiratoire 3514 pour un réservoir de gaz expiratoire 3511 au repos est ici maximale. En cas d'apport de gaz par le premier canal de collecte 3513, le premier réservoir de gaz expiratoire 3511 a tendance à se remplir, c'est-à-dire se gonfler, jusqu'à ce que les pressions régnant dans le premier réservoir de gaz expiratoire 3511 et le premier volume interne expiratoire 3512 (et donc la première sortie de gaz 3515) s'équilibrent. Ainsi, si l'apport de gaz par le premier canal de collecte 3513 est un volume V1, alors le même volume V1 sera évacué du premier volume interne expiratoire 3512 par la première sortie de gaz 3515. Il s'ensuit alors que, plus le premier réservoir de gaz expiratoire 3511 se remplit, plus la distance retournée par le premier capteur de distance expiratoire 3514 diminue.

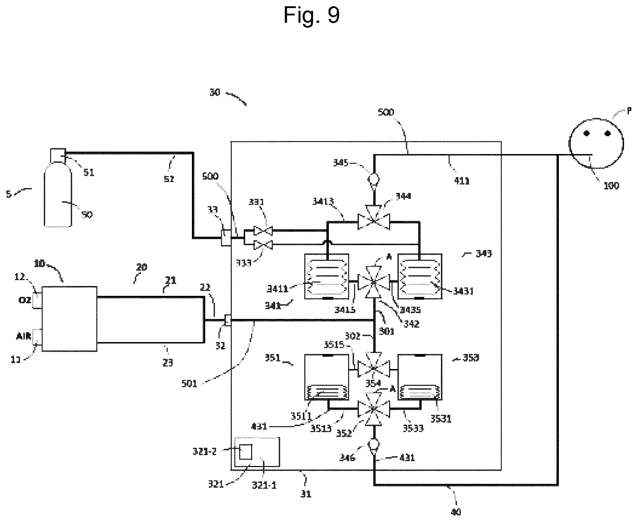

Fig. 9 à Fig. 13 illustrent alors le fonctionnement de l'interface de ventilation 30 couplée à ventilateur médical 10 et par ailleurs à une source de gaz thérapeutique 5, par exemple une bouteille de gaz 50 contenant un mélange gazeux sous pression binaire formé de 60% Ar et de 40% O₂ (% vol).

Plus précisément, Fig. 9 illustre l'interface de ventilation 30 dans sa configuration initiale. Elle est connectée, via le second port d'entrée 33 et le flexible de raccordement 52 à la source de gaz thérapeutique 5 et, via le premier port d'entrée 32, au ventilateur médical 10, tel celui de la Fig. 1, via le circuit patient 20.

Un utilisateur peut régler les paramètres de ventilation de manière à ce que le ventilateur 10 délivre un volume gazeux de 500 mL à une fréquence respiratoire de 15 coups par minute (c/min) par exemple.

A cet instant, les première et seconde vannes d'admission 331, 333, préférentiellement identiques l'une à l'autre, sont en position fermée.

Le premier réservoir de gaz inspiratoire 3411 de la première chambre inspiratoire 341 est entièrement rempli par le gaz thérapeutique. Une seconde chambre inspiratoire 343, identique à la première chambre inspiratoire 341, est dans une configuration similaire, c'est-à-dire qu'elle comprend un second réservoir de gaz inspiratoire 3431, qui est aussi entièrement rempli par du gaz thérapeutique provenant de la source de gaz 5.

La première vanne 344 réalise une connexion fluidique entre le premier canal d'évacuation 3413 et une ligne d'administration de gaz 411 dans laquelle est agencée une valve anti-retour 345 ne permettant au gaz de circuler que dans un sens, à savoir du premier canal d'évacuation 3413 vers la ligne d'administration de gaz 411 mais pas dans le sens inverse.

Le premier sélecteur 342 réalise une connexion fluidique entre, d'une part, un conduit inspiratoire 301 et la première entrée de gaz 3415 et, d'autre part, une seconde entrée de gaz 3435 et l'ambient A.

Le premier réservoir de gaz expiratoire 3511 de la première chambre expiratoire 351 est entièrement dégonflé. Une seconde chambre expiratoire 353, identique à la première chambre expiratoire 351 est dans une configuration similaire, c'est-à-dire qu'elle renferme un second réservoir de gaz expiratoire 3531 entièrement dégonflé.

La seconde vanne 354 réalise une connexion fluidique entre la première sortie de gaz 3515 et un conduit expiratoire 302.

Le second sélecteur 352 réalise une connexion fluidique entre, d'une part, une ligne expiratoire 431, dans laquelle est agencée une seconde valve anti-retour 346, et le premier canal de collecte 3513 et, d'autre part, un second canal de collecte 3533 et l'ambient A. La seconde valve anti-retour 356 empêche toute circulation inverse de gaz, c'est-à-dire du premier canal de collecte 3513 vers la ligne expiratoire 431 par exemple.

Autrement dit, la ligne expiratoire 431 se ramifie en le premier canal de collecte 3513 et le second canal de collecte 3533.

A cet instant, le ventilateur 10 délivre une phase inspiratoire sous la forme d'un volume de gaz, ici 500 ml. Comme décrit en Fig. 1, ce volume de gaz va se diriger dans la branche inspiratoire 21 du circuit patient 20 pour atteindre la pièce Y 22 et l'entrée de ventilation 32 de l'interface ventilatoire 30, c'est-à-dire qu'aucune portion de ce gaz ne se propage dans la branche expiratoire 23 du circuit patient 20.

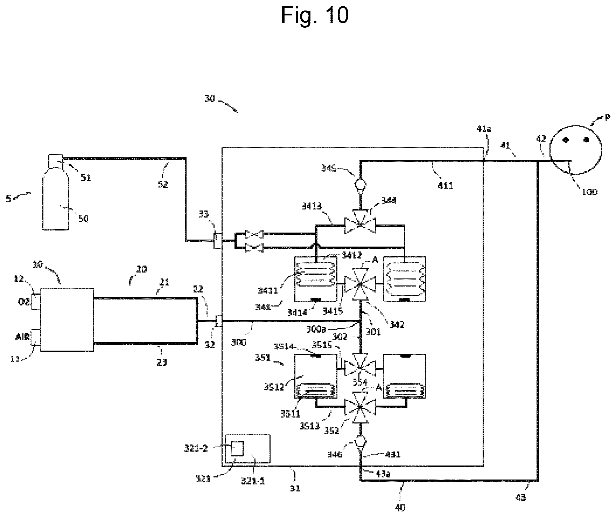

Comme illustré sur Fig. 10, un conduit commun 300 prolonge l'entrée de ventilation 32 et permet au gaz de circuler à l'intérieur même de l'interface de ventilation 30. Ce conduit commun 300 se termine à un embranchement 300a pour être prolongé par les conduits inspiratoire 301 et expiratoire 302, respectivement.

Etant donné la configuration du premier sélecteur 342, le gaz va être dirigé dans la première entrée de gaz 3415 pour entrer dans le premier volume interne inspiratoire 3412 de la première chambre inspiratoire 341. Puisque dans le même temps, la première vanne 344 réalise une connexion fluidique entre le premier canal d'évacuation 3413 et la ligne d'administration 411. Le premier réservoir de gaz inspiratoire 3411 va se déformer d'autant et expulser le volume de gaz à travers le premier canal d'évacuation 3413, puis la ligne d'administration 411. Cette diminution de volume du premier réservoir de gaz inspiratoire 3411 est détectée par le premier capteur de distance inspiratoire 3414, via l'augmentation de la distance « d » mesurée par ce premier capteur 3414. Cette augmentation de distance « d » informe les moyens de pilotage 321 que le système d'interfaçage 3 est en train de délivrer du gaz, c'est-à-dire qu'il est en phase inspiratoire. Le volume de gaz entrant dans le premier volume interne 3412 est le gaz issu du ventilateur 10 constitué d'un mélange d'air et d'O₂ alors que le volume de gaz sortant du premier réservoir de gaz inspiratoire 3411 est le gaz thérapeutique (e.g. Ar/O2) provenant de la source de gaz thérapeutique 5.

En se référant toujours à la même figure, la ligne d'administration est connectée en 41a a la branche inspiratoire 41 d'un second circuit patient 40 auquel est connecte le patient P via son interface respiratoire 100 et une pièce Y 42. Le circuit patient 40 comprend en outre une branche expiratoire 43, raccordée en 43a à la ligne expiratoire 431 via l'un des ports de raccordement 34, 35 de l'interface ventilatoire 30.

Du fait de la position de la seconde vanne 354, il existe une connexion fluidique entre le conduit expiratoire 302 et la première sortie de gaz 3515, et donc avec le premier volume interne expiratoire 3512 de la première chambre expiratoire 351. Par ailleurs, le second sélecteur 352 établit une autre connexion fluidique entre la ligne expiratoire 431 et le premier canal de collecte 3513, et par prolongement le premier réservoir de gaz expiratoire 3511.

Comme le ventilateur 10 est en train de réaliser une phase inspiratoire, le gaz ne peut circuler dans le conduit expiratoire 302, et par conséquent s'échapper du premier volume interne expiratoire 3512. Dès lors, le premier réservoir de gaz expiratoire 3511 ne peut se déformer, i.e. se remplir de gaz provenant de la ligne expiratoire 431 et ainsi, de la branche expiratoire 43 du circuit patient P. Ainsi, le volume de gaz circulant dans le premier canal d'évacuation 3413 et la ligne d'administration 411 n'a d'autre possibilité que de se propager dans la branche inspiratoire 41 puis la pièce Y 42 du second circuit patient 40, pour ensuite être délivré au patient P, via l'interface respiratoire 100, i.e. un masque ou analogue. Cette délivrance de gaz, i.e. de volume gazeux ici, associée aux caractéristiques physiologiques du patient, entraine une élévation de pression dans le circuit patient 40 et des connexions fluidiques de l'interface ventilatoire 30 à laquelle le circuit patient 40 est relié.

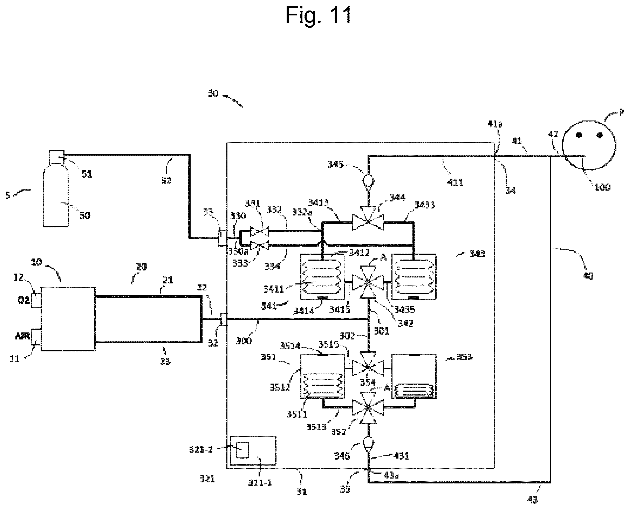

Après avoir délivré la phase inspiratoire, le ventilateur 10 bascule en phase expiratoire et ouvre la branche expiratoire 23 en bloquant par ailleurs la branche inspiratoire 21 du premier circuit patient 20. Cette phase expiratoire est illustrée en Fig. 11.

Il apparaît alors une dépressurisation des éléments situés en aval du circuit patient 20, en particulier le conduit commun 300 et les conduits inspiratoire 301 et expiratoire 302. La première entrée de gaz 3415 et le premier volume interne inspiratoire 3412 de la première chambre inspiratoire 341, en relation fluidique avec le conduit inspiratoire 302, vont également subir cette dépressurisation.

Similairement, la configuration initiale de la seconde vanne 354 assure la dépressurisation de la première sortie de gaz 3515 et du premier volume interne expiratoire 3512 de la première chambre expiratoire 351, ces derniers étant en relation fluidique avec le conduit expiratoire 302.

Alors que la pression régnant dans le second circuit patient 40 devient supérieure à la pression régnant dans les éléments susmentionnés, le patient P est libre d'expirer dans l'interface respiratoire 100. Du fait des valves anti-retour 345 et 346, ce gaz ne peut emprunter qu'un seul chemin, à savoir passer par la pièce Y 42 et la branche expiratoire 43 du second circuit patient 40, prolongée par la ligne expiratoire 431.

Par le biais de la configuration du second sélecteur 352, le gaz est dirigé dans le premier canal de collecte 3513 puis le premier réservoir de gaz expiratoire 3511 de la première chambre expiratoire 351. Du fait de la moindre pression régnant dans le premier volume interne expiratoire 3512, le premier réservoir de gaz expiratoire va se déformer, i.e. se remplir des gaz expirés par le patient P, et chasser d'autant l'air du premier volume interne expiratoire 3512, qui va successivement emprunter la première sortie de gaz 3515, le conduit expiratoire 302 et conduit commun 300.

Ce gaz sera ensuite dirigé vers la branche expiratoire 23 du circuit patient 20 (via l'entrée de ventilation 32 et la pièce Y 22) pour être évacué par le ventilateur 1, ayant au préalable mesuré ce volume expiré.

En d'autres termes, en considérant que l'interface respiratoire 100 du patient réalise une étanchéité adéquate, le volume V délivré pendant la phase inspiratoire, e.g.500 ml, se retrouve collecté et expiré par la première chambre expiratoire 351

Le volume de gaz sortant du premier volume interne expiratoire 3512 est le gaz issu du ventilateur 10 (i.e. mélange air/O₂), alors que le volume de gaz collecté dans le premier réservoir de gaz expiratoire 3511 est le gaz thérapeutique (e.g. Ar/O₂).

L'interface ventilatoire 30 opère une séparation parfaite entre d'une part, le mélange de gaz provenant du ventilateur 10, pour lequel ledit ventilateur 10 a été conçu et validé, et le gaz thérapeutique inhalé par le patient P, alors même que le ventilateur 10 détermine, via ses paramètres de ventilation, la façon dont l'aide respiratoire est délivrée au patient P.

Alors que le patient est en train d'expirer dans le premier réservoir de gaz expiratoire 3511, le premier capteur de distance expiratoire 3514 va informer les moyens de pilotage 321 qu'une expiration est en cours. En effet, au fur et à mesure que le premier réservoir de gaz expiratoire 3511 se rempli, la distance « d » mesurée par le premier capteur de distance expiratoire 3514 va diminuer, renseignant alors qu'une phase expiratoire est en cours.

Les moyens de pilotage 321 pilotent alors une modification de la configuration de la première vanne 344 et du premier sélecteur 342 de 'interface ventilatoire 30.

Plus précisément, la première vanne 344 est commandée pour réaliser une connexion fluidique entre un second canal d'évacuation 3433 faisant partie de la seconde chambre inspiratoire 343, et la ligne d'administration de gaz 411.

Par ailleurs, le premier sélecteur 342 réalise une connexion fluidique entre, d'une part, le conduit inspiratoire 301 et une seconde entrée de gaz 3435 faisant partie de la seconde chambre inspiratoire 343 et, d'autre part, la première entrée de gaz 3415 et l'atmosphère ambiante A.

Ainsi, la première chambre inspiratoire 341 se retrouve complètement isolée du patient et du ventilateur 10. Le premier volume interne inspiratoire 3412 est, par sa connexion fluidique avec la première entrée de gaz 3415, mis à la pression atmosphérique ambiante, et le premier canal d'évacuation 3413, donc le premier réservoir de gaz inspiratoire 3411, est isolé de la ligne d'administration 411 du fait de la configuration de la première vanne 344.

Une tel isolement de la première chambre inspiratoire 341 permet aux moyens de pilotage 321 de procéder au remplissage du premier réservoir de gaz inspiratoire 3411 par le gaz thérapeutique issu de la source 5. En effet, comme décrit précédemment, la source de gaz thérapeutique 5 est connectée, via son flexible de raccordement 52, à l'entrée de gaz 33 de l'interface ventilatoire 30.

L'entrée de gaz 33 de l'interface ventilatoire 30 est reliée via la ligne d'acheminement de gaz additionnel 500 au premier port de raccordement 34.

La ligne d'acheminement de gaz additionnel 500 comprend, en aval de l'entrée de gaz 33, un premier tronçon ou ligne d'admission commune 330 qui se sépare à un embranchement 330a en deux lignes d'admission distinctes formant deux tronçons parallèles, à savoir une première ligne d'admission 332 dans laquelle est agencée une première vanne d'admission 331 et une seconde ligne d'admission 334 dans laquelle est agencée une seconde vanne d'admission 333.

Il apparait que la première ligne d'admission 332 est reliée fluidiquement au premier canal d'évacuation 3413 à un embranchement 332a, et donc ainsi au premier réservoir de gaz inspiratoire 3411. Dans cette configuration, les moyens de pilotage 321 déterminent d'ouvrir la première vanne d'admission 331, ce qui va permettre au gaz issu de la source de gaz thérapeutique 5 d'emprunter successivement la ligne d'admission commune 330, la première ligne d'admission 332 et le premier canal d'évacuation 3413 pour ensuite entrer dans le premier réservoir de gaz inspiratoire 3411.

Devant cet apport de gaz, le premier réservoir de gaz inspiratoire 3411 va se gonfler et chasser le mélange d'air et d'O₂ contenu dans le premier volume interne inspiratoire 3412 à l'ambient, via la première entrée de gaz 3415. Durant ce processus, le premier capteur de distance inspiratoire 3414 renvoie une mesure de distance « d » diminuant au fur et à mesure que le premier réservoir de gaz inspiratoire se remplit.

Cette mesure de distance « d » va diminuer jusqu'à atteindre la valeur D_{REPOS} correspondant à un volume maximal renfermé par le premier réservoir de gaz inspiratoire 3411. Lorsqu'une telle condition est vérifiée, les moyens de pilotage 321 déterminent de couper l'arrivée de gaz thérapeutique en fermant la première vanne d'admission 331. Ainsi, la première chambre inspiratoire 341 revient alors dans une configuration initiale ou son premier réservoir de gaz inspiratoire 3411 est entièrement rempli et prêt à l'emploi.

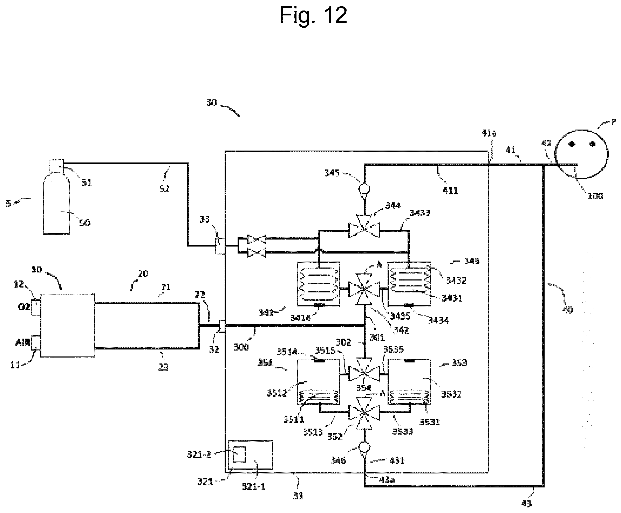

Selon les paramètres ventilatoires réglés sur le ventilateur 10, celui-ci va interrompre la phase expiratoire pour délivrer une nouvelle phase inspiratoire comme illustré en Fig. 12.

A l'inverse de la configuration initiale décrite en Fig. 9, c'est au tour de la seconde chambre inspiratoire 343 de fournir le gaz thérapeutique au patient. A cet effet, le gaz fourni par le ventilateur 10, se propageant dans le conduit principal 300 et conduit inspiratoire 301, va être dirigé dans la seconde entrée de gaz 3435 pour entrer dans un second volume interne inspiratoire 3432 de la seconde chambre inspiratoire 343.

Etant donné que, dans le même temps, la première vanne 344 réalise une connexion fluidique entre le second canal d'évacuation 3433 et la ligne d'administration 411, le second réservoir de gaz inspiratoire 3431 va se déformer d'autant et expulser le volume de gaz à travers le second canal d'évacuation 3433. Cette diminution de volume du second réservoir de gaz inspiratoire 3431 est détecté par un second capteur de distance inspiratoire 3434, similaire en tout point au premier capteur de distance 3414.

Comme expliqué en références aux Fig. 5 à Fig. 7, le second capteur de distance inspiratoire 3434 est capable de mesurer le taux de gonflage du second réservoir de gaz inspiratoire 3431, via des mesures successives de distance « d » et d'informer les moyens de pilotage 321 que l'interface ventilatoire 30 est en train de délivrer du gaz, c'est-à-dire qu'elle est en phase inspiratoire.

Comme le ventilateur 10 est en phase inspiratoire, aucun gaz ne peut circuler dans le conduit expiratoire 302, ou plus particulièrement en provenance du premier volume interne expiratoire 3512. La totalité du volume gazeux issu du second réservoir de gaz inspiratoire 3431 est dirigé vers le patient en empruntant la ligne d'administration 411, la branche inspiratoire 41 et la pièce Y 43 du circuit patient 4 et, finalement, l'interface respiratoire 100.

Au vu du déroulement de la séquence de pilotage de l'interface ventilatoire 30, dans une telle situation d'administration de gaz au patient P, les moyens de pilotage 321 déterminent qu'il convient alors de « purger » la première chambre expiratoire 351, étant donné que le premier réservoir de gaz expiratoire 3511 est rempli du gaz précédemment expiré par le patient.

A cet effet, la seconde vanne 354 est pilotée par lesdits moyens de pilotage 321 pour mettre en relation fluidique le conduit expiratoire 302 avec une seconde sortie de gaz 3535, qui est fluidiquement reliée à un second volume interne expiratoire 3532 de la seconde chambre expiratoire 353. Les éléments constitutifs de la première chambre expiratoire 351, en particulier le premier volume interne expiratoire 3512 et première sortie de gaz 3515 deviennent alors isolés du conduit expiratoire 302.

Par ailleurs, les moyens de pilotage 321 commandent dans le même temps le second sélecteur 352 de manière à ce qu'une connexion fluidique soit réalisée entre, d'une part, le premier canal de collecte 3513 et l'atmosphère ambiante et, d'autre part, la ligne expiratoire 431 et le second canal de collecte 3533. Ce changement de configuration du second sélecteur 352 isole alors le premier réservoir de gaz expiratoire 3511 et le place aux conditions de pression atmosphérique, en réponse à la connexion fluidique réalisée par le second sélecteur 352.

Dès lors, rien ne s'oppose à ce que le premier réservoir de gaz expiratoire 3511 se vide de son contenant, i.e. des gaz précédemment expirés par le patient, et les évacue à l'atmosphère. Le premier capteur de distance expiratoire 3514, qui mesure le retour à une position initiale de la première chambre expiratoire 351, est en capacité de confirmer aux moyens de pilotage 321 que la distance mesurée est redevenue maximale, signe que le premier réservoir de gaz expiratoire 3511 est entièrement dégonflé.

Dans le même temps, ce changement de configuration du second sélecteur 352 met en relation la ligne expiratoire 431 avec le second canal de collecte de gaz 3533, relié fluidiquement au second réservoir de gaz expiratoire 3531. Toujours est-il que la relation fluidique entre la seconde sortie de gaz 3535 avec le conduit expiratoire 302, alors que le ventilateur 10 est en phase inspiratoire, va interdire au gaz d'être expulsé du second volume interne expiratoire 3532 et donc, par conséquent, au gaz de circuler dans la branche expiratoire 43 du circuit patient 4 et la ligne expiratoire 431 pour venir remplir le second réservoir de gaz expiratoire 3531 via le second canal de collecte 3513: ce changement de configuration ne remet en cause le fait que le patient P recevra le volume de gaz insufflé par le ventilateur 1.

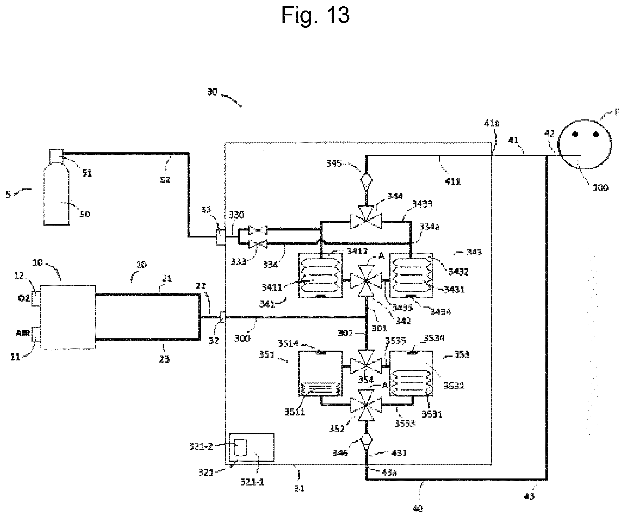

Lorsque le ventilateur 10 détermine un nouveau passage en expiration, comme illustré en Fig. 13, la seconde chambre expiratoire 353 est en mesure de collecter les gaz expirés par le patient, via le changement de configuration de la seconde vanne 354 et du second sélecteur 352, et a première chambre inspiratoire 341 est dans sa position de repos, c'est-à-dire que le premier réservoir de gaz inspiratoire 3411 est entièrement rempli de gaz thérapeutique et prêt à fournir une nouvelle phase inspiratoire au patient. De plus, la première chambre expiratoire 351 est dans une position de repos, c'est-à-dire que le premier réservoir de gaz expiratoire 3511 est entièrement vide et prêt à collecter, le moment venu, une nouvelle expiration du patient, alors que la seconde chambre inspiratoire 343, en particulier son second réservoir de gaz inspiratoire 3431 est partiellement vidé du gaz thérapeutique, du fait de l'insufflation précédente.

Suivant la même logique, les gaz expirés par le patient P seront alors dirigés dans le second réservoir de gaz expiratoire 3531 via le second canal de collecte 3533. Ceci déplace mécaniquement le même volume du second volume interne expiratoire 3532, qui circule alors de la seconde sortie de gaz 3535 vers la branche expiratoire 23 du premier circuit patient 20, afin d'être analysé par le ventilateur 10.

Par ailleurs, un second capteur de distance expiratoire 3534, identique dans son positionnement et son fonctionnement au premier capteur de distance expiratoire 3514 réalise une série de mesure permettant d'indiquer aux moyens de pilotage 321 qu'une expiration est cours dans la seconde chambre expiratoire 353.

Ainsi, les moyens de pilotage 321 agissent à nouveau sur la première vanne 344 et le premier sélecteur 342 pour les placer dans la même configuration que celle décrite en Fig. 9, c'est-à-dire que la première chambre inspiratoire 341 deviendra la prochaine source de gaz thérapeutique pour le patient P lors de la prochaine phase inspiratoire, dictée par le ventilateur 10.

Selon cette configuration, la seconde chambre inspiratoire se retrouve isolée. Le second volume interne inspiratoire 3432 est connecté fluidiquement à l'atmosphère ambiante (en A) et le second canal d'évacuation 3433 est isolé de la ligne d'administration 411. Ceci permet de remplir le second réservoir de gaz inspiratoire 3431 de gaz thérapeutique pour le remettre dans une configuration de repos. Les moyens de pilotage 321 commandent alors la seconde vanne d'admission 333 de manière à ce que le gaz issu de la source de gaz thérapeutique 5 emprunte la ligne d'admission commune 330, puis la seconde ligne d'admission 334 pour être dirigé, à un embranchement 334a, dans le second conduit d'évacuation 3433 et finalement le second réservoir de gaz inspiratoire 3431.

Devant cet apport de gaz, le second réservoir de gaz inspiratoire 3431 va se gonfler et chasser le mélange d'air et d'O₂ contenu dans le second volume interne inspiratoire 3432 à l'ambiante, via la seconde entrée de gaz 3435. Durant ce processus, le second capteur de distance inspiratoire 3434 renvoie une mesure de distance « d » diminuant au fur et à mesure que le premier réservoir de gaz inspiratoire se remplit.

Cette mesure de distance « d » va diminuer jusqu'à atteindre la valeur D_{REPOS} correspondant à un volume maximal renfermé par le second réservoir de gaz inspiratoire 3431. Lorsqu'une telle condition est vérifiée, les moyens de pilotage 321 déterminent de couper l'arrivée de gaz thérapeutique en fermant la seconde vanne d'admission 333. Ainsi, la seconde chambre inspiratoire 343 revient alors également dans une configuration initiale ou le second réservoir de gaz inspiratoire 3431 est entièrement rempli et prêt à l'emploi.

A la prochaine inspiration, c'est la première chambre inspiratoire 341 qui fournit alors le gaz au patient. Dans le même temps, les moyens de pilotage 321 sélectionnent la première chambre expiratoire 351 comme futur organe de collecte des gaz expirés, tandis que la seconde chambre expiratoire 353 se « purge » de manière à revenir dans une position de repos.

Par le biais de ce fonctionnement cyclique entre les première et seconde chambre inspiratoire 341, 343, et les première et seconde chambre expiratoire 351, 353, l'interface ventilatoire 30 peut délivrer un gaz thérapeutique au patient P sans impacter le fonctionnement du ventilateur médical 10, c'est-à-dire que celui-ci conserve sa capacité à assurer les paramètres de ventilation réglés, même si ce ventilateur médical 10 n'est pas conçu ou adapté pour délivrer le mélange gazeux provenant de la source 5, à savoir ici le mélange Art/O₂.

De manière générale, dans le mode de réalisation proposé, on utilise des capteurs de distance pour déterminer la phase dans laquelle se trouve l'interface ventilatoire 30, c'est-à-dire en cours d'une inspiration ou bien d'une expiration, et quelles chambres doivent revenir en position initiale, c'est-à-dire remplissage pour les chambres inspiratoires ou purge pour les chambres expiratoires. Dans d'autres modes de réalisation, on peut remplacer ces capteurs de distance par d'autres moyens de mesure équivalents, comme des capteurs de niveaux, de position ou de débit, adaptés pour déterminer le degré de déformation des différents réservoirs.

## Revendications

1. Interface de ventilation (30) comprenant au moins :
- une ligne d'acheminement de gaz principal (501) comprenant un premier port d'entrée de gaz (32) pour recevoir du gaz fourni par un ventilateur médical (10) ;
- une ligne d'acheminement de gaz additionnel (500) comprenant un second port d'entrée de gaz (33) pour recevoir du gaz fourni par une source de gaz additionnelle (5) ; et
- une ligne expiratoire (431) ;
- la ligne d'acheminement de gaz additionnel (500) comprenant une première chambre inspiratoire (341) et une seconde chambre inspiratoire (343) agencées en parallèle l'une de l'autre ;
- la première chambre inspiratoire (341) contenant un premier réservoir inspiratoire (3411) à volume interne variable agencé au sein d'une première enceinte inspiratoire, le volume interne variable du premier réservoir inspiratoire (3411) étant en communication fluidique avec la ligne d'acheminement de gaz additionnel (500), et
- la seconde chambre inspiratoire (343) contenant un second réservoir inspiratoire (3431) à volume interne variable agencé au sein d'une seconde enceinte inspiratoire, le volume interne variable du second réservoir inspiratoire (3431) étant en communication fluidique avec la ligne d'acheminement de gaz additionnel (500)
- la ligne expiratoire (431) comprenant une première chambre expiratoire (351) et une seconde chambre expiratoire (353) agencées en parallèle l'une de l'autre ;
- la première chambre expiratoire (351) contenant un premier réservoir expiratoire (3511) à volume interne variable agencé au sein d'une première enceinte expiratoire, le volume interne variable du premier réservoir expiratoire (3511) étant en communication fluidique avec la ligne expiratoire (431) ; et
- la seconde chambre expiratoire (353) comprenant un second réservoir expiratoire (3531) à volume interne variable agencé au sein d'une seconde enceinte expiratoire, le volume interne variable du second réservoir expiratoire (3531) étant en communication fluidique avec la ligne expiratoire (431) ; et
- la ligne d'acheminement de gaz principal (501) étant en communication fluidique avec les volumes internes des premières et secondes enceintes inspiratoires et expiratoires.

2. Interface selon la revendication 1, **caractérisée en ce que** la ligne d'acheminement de gaz additionnel (500) se ramifie en un premier tronçon de ligne (330) et un second tronçon de ligne (332), le premier tronçon de ligne (330) comprenant la première chambre inspiratoire (341) et le second tronçon de ligne (332) comprenant la seconde chambre inspiratoire (343).

3. Interface selon la revendication 2, **caractérisée en ce que** le premier tronçon de ligne (330) comprend une première vanne d'admission (331) de gaz et le second tronçon de ligne (332) comprend une seconde vanne d'admission (333) de gaz.

4. Interface selon la revendication 1, **caractérisée en ce que** la ligne expiratoire (431) se ramifie en un premier canal de collecte (3513) et un second canal de collecte (3533), le premier canal de collecte (3513) comprenant la première chambre expiratoire (351) et le second canal de collecte (3533) comprenant la seconde chambre expiratoire (353).

5. Interface selon l'une des revendications précédentes, **caractérisée en ce que** :
- la première chambre inspiratoire (341) comprend un premier capteur inspiratoire (3414), et la seconde chambre inspiratoire (343) comprend un second capteur inspiratoire (3434), et
- la première chambre expiratoire (351) comprend un premier capteur expiratoire (3514), et la seconde chambre expiratoire (353) comprend un second capteur expiratoire (3534).

6. Interface selon l'une des revendications précédentes, **caractérisée en ce qu'**elle comprend des moyens de pilotage (321).

7. Interface selon les revendications 3 et 6, **caractérisée en ce que** les moyens de pilotage (321) pilotent au moins les première et seconde vannes d'admission de gaz (331, 333).

8. Interface selon les revendication 5 et 6, **caractérisée en ce que** les premier et second capteurs inspiratoires et expiratoires (3414, 3434 ; 3514, 3534) sont reliés électriquement aux moyens de pilotage (321).

9. Interface selon la revendication 1, **caractérisée en ce que** le premier et/ou le second réservoir inspiratoire (3411, 3431) et/ou le premier et/ou le second réservoir expiratoire (3511, 3531) comprennent des soufflets, de préférence des soufflets circulaires.

10. Interface selon la revendication 6, **caractérisée en ce que** les moyens de pilotage (321) comprennent au moins une carte de commande électronique et/ou au moins une unité de contrôle à microprocesseur.

11. Interface selon la revendication 1, **caractérisée en ce que** le premier et/ou le second réservoir inspiratoire (3411, 3431) et/ou le premier et/ou le second réservoir expiratoire (3511, 3531) sont formés d'au moins un matériau souple, de préférence de silicone de grade médical.

12. Installation de ventilation assistée (5, 10, 30) d'un patient comprenant :
- une interface de ventilation (30) selon l'une des revendications précédentes,
- un ventilateur médical (10) relié fluidiquement au premier port d'entrée de gaz (32) de la ligne d'acheminement de gaz principal (501), via un premier circuit patient (20), et
- une source de gaz additionnelle (5) reliée fluidiquement au second port d'entrée de gaz (33) de la ligne d'acheminement de gaz additionnel (500), via un flexible de raccordement (52).

13. Installation de ventilation assistée selon la revendication 12, **caractérisée en ce que** la source de gaz additionnelle (5) est une bouteille de gaz sous pression contenant un gaz ou mélange gazeux à base d'argon, en particulier un mélange argon/O₂, ou à base de xénon, en particulier un mélange xénon/azote/O₂.

## Patentansprüche

1. Beatmungsschnittstelle (30), umfassend mindestens:
- eine Hauptgas-Zuführungsleitung (501), umfassend einen ersten Gaseinlassanschluss (32) zum Aufnehmen des von einem medizinischen Beatmungsgerät (10) abgegebenen Gases;
- eine Zusatzgas-Zuführungsleitung (500), umfassend einen zweiten Gaseinlassanschluss (33) zum Aufnehmen des von einer zusätzlichen Gasquelle (5) abgegebenen Gases; und
- eine Ausatemleitung (431);
- wobei die Zusatzgas-Zuführungsleitung (500) eine erste Einatemkammer (341) und eine zweite Einatemkammer (343) umfasst, die parallel zueinander angeordnet sind;
- wobei die erste Einatemkammer (341) einen ersten Einatembehälter (3411) mit variablem Innenvolumen enthält, der innerhalb eines ersten Einatemgehäuses angeordnet ist, wobei das variable Innenvolumen des ersten Einatembehälters (3411) in Fluidverbindung mit der Zusatzgas-Zuführungsleitung (500) steht, und
- wobei die zweite Einatemkammer (343) einen zweiten Einatembehälter (3431) mit variablem Innenvolumen enthält, der innerhalb eines zweiten Einatemgehäuses angeordnet ist, wobei das variable Innenvolumen des zweiten Einatembehälters (3431) in Fluidverbindung mit der Zusatzgas-Zuführungsleitung (500) steht,
- wobei die Ausatemleitung (431) eine erste Ausatemkammer (351) und eine zweite Ausatemkammer (353) umfasst, die parallel zueinander angeordnet sind;
- wobei die erste Ausatemkammer (351) einen ersten Ausatembehälter (3511) mit variablem Innenvolumen enthält, der innerhalb eines ersten Ausatemgehäuses angeordnet ist, wobei das variable Innenvolumen des ersten Ausatembehälters (3511) in Fluidverbindung mit der Ausatemleitung (431) steht; und
- wobei die zweite Ausatemkammer (353) einen zweiten Ausatembehälter (3531) mit variablem Innenvolumen umfasst, der innerhalb eines zweiten Ausatemgehäuses angeordnet ist, wobei das variable Innenvolumen des zweiten Ausatembehälters (3531) in Fluidverbindung mit der Ausatemleitung (431) steht; und
- wobei die Hauptgas-Zuführungsleitung (501) in Fluidverbindung mit den Innenvolumina der ersten und zweiten Einatem- und Ausatemgehäuse steht.

2. Schnittstelle nach Anspruch 1, **dadurch gekennzeichnet, dass** sich die Zusatzgas-Zuführungsleitung (500) in einen ersten Leitungsabschnitt (330) und einen zweiten Leitungsabschnitt (332) verzweigt, wobei der erste Leitungsabschnitt (330) die erste Einatemkammer (341) umfasst und wobei der zweite Leitungsabschnitt (332) die zweite Einatemkammer (343) umfasst.

3. Schnittstelle nach Anspruch 2, **dadurch gekennzeichnet, dass** der erste Leitungsabschnitt (330) ein erstes Gaseinlassventil (331) umfasst und der zweite Leitungsabschnitt (332) ein zweites Gaseinlassventil (333) umfasst.

4. Schnittstelle nach Anspruch 1, **dadurch gekennzeichnet, dass** sich die Ausatemleitung (431) in einen ersten Sammelkanal (3513) und einen zweiten Sammelkanal (3533) verzweigt, wobei der erste Sammelkanal (3513) die erste Ausatemkammer (351) umfasst und wobei der zweite Sammelkanal (3533) die zweite Ausatemkammer (353) umfasst.

5. Schnittstelle nach einem der vorhergehenden Ansprüche, **dadurch gekennzeichnet, dass**:
- die erste Einatemkammer (341) einen ersten Einatemsensor (3414) umfasst und die zweite Einatemkammer (343) einen zweiten Einatemsensor (3434) umfasst, und
- die erste Ausatemkammer (351) einen ersten Ausatemsensor (3514) umfasst und die zweite Ausatemkammer (353) einen zweiten Ausatemsensor (3534) umfasst.

6. Schnittstelle nach einem der vorhergehenden Ansprüche, **dadurch gekennzeichnet, dass** sie Ansteuerungsmittel (321) umfasst.

7. Schnittstelle nach den Ansprüchen 3 und 6, **dadurch gekennzeichnet, dass** die Ansteuerungsmittel (321) mindestens die ersten und zweiten Gaseinlassventile (331, 333) ansteuern.

8. Schnittstelle nach den Ansprüchen 5 und 6, **dadurch gekennzeichnet, dass** die ersten und zweiten Einatem- und Ausatemsensoren (3414, 3434; 3514, 3534) mit den Ansteuerungsmitteln (321) elektrisch verbunden sind.

9. Schnittstelle nach Anspruch 1, **dadurch gekennzeichnet, dass** der erste und/oder der zweite Einatembehälter (3411, 3431) und/oder der erste und/oder der zweite Ausatembehälter (3511, 3531) Faltenbalge, bevorzugt runde Faltenbalge, umfassen.

10. Schnittstelle nach Anspruch 6, **dadurch gekennzeichnet, dass** die Ansteuerungsmittel (321) mindestens eine elektronische Steuerkarte und/oder mindestens eine Steuerungseinheit mit Mikroprozessor umfassen.

11. Schnittstelle nach Anspruch 1, **dadurch gekennzeichnet, dass** der erste und/oder der zweite Einatembehälter (3411, 3431) und/oder der erste und/oder der zweite Ausatembehälter (3511, 3531) aus einem weichen Material, bevorzugt aus Silikon in Medizinqualität, gebildet sind.

12. Anlage zur assistierten Beatmung (5, 10, 30) eines Patienten, umfassend:
- eine Beatmungsschnittstelle (30) nach einem der vorhergehenden Ansprüche,
- ein medizinisches Beatmungsgerät (10), das fluidisch mit dem ersten Gaseinlassanschluss (32) der Hauptgas-Zuführungsleitung (501), über einen ersten Patientenkreis (20), verbunden ist, und
- eine zusätzliche Gasquelle (5), die fluidisch mit dem zweiten Gaseinlassanschluss (33) der Zusatzgas-Zuführungsleitung (500), über einen Anschlussschlauch (52), verbunden ist.

13. Anlage zur assistierten Beatmung nach Anspruch 12, **dadurch gekennzeichnet, dass** die zusätzliche Gasquelle (5) eine Druckgasflasche ist, die ein Gas oder Gasgemisch auf Basis von Argon, insbesondere ein Argon/O₂-Gemisch, oder auf Basis von Xenon, insbesondere ein Xenon/Stickstoff/O₂-Gemisch, enthält.

## Claims

1. Ventilation interface (30) comprising at least:
- a main gas supply line (501) comprising a first gas inlet port (32) for receiving gas delivered by a medical ventilator (10);
- an additional gas supply line (500) comprising a second gas inlet port (33) for receiving gas delivered by an additional gas source (5); and
- an expiratory line (431);
- the additional gas supply line (500) comprising a first inspiratory chamber (341) and a second inspiratory chamber (343) arranged in parallel with each other;
- the first inspiratory chamber (341) containing a first inspiratory reservoir (3411) of variable internal volume arranged within a first inspiratory enclosure, the variable internal volume of the first inspiratory reservoir (3411) being in fluidic communication with the additional gas supply line (500), and
- the second inspiratory chamber (343) containing a second inspiratory reservoir (3431) of variable internal volume arranged within a second inspiratory enclosure, the variable internal volume of the second inspiratory reservoir (3431) being in fluidic communication with the additional gas supply line (500),
- the expiratory line (431) comprising a first expiratory chamber (351) and a second expiratory chamber (353) arranged in parallel with each other;
- the first expiratory chamber (351) containing a first expiratory reservoir (3511) of variable internal volume arranged within a first expiratory enclosure, the variable internal volume of the first expiratory reservoir (3511) being in fluidic communication with the expiratory line (431); and
- the second expiratory chamber (353) comprising a second expiratory reservoir (3531) of variable internal volume arranged within a second expiratory enclosure, the variable internal volume of the second expiratory reservoir (3531) being in fluidic communication with the expiratory line (431); and
- the main gas supply line (501) being in fluidic communication with the internal volumes of the first and second inspiratory and expiratory enclosures.

2. Interface according to Claim 1, **characterized in that** the additional gas supply line (500) branches into a first line section (330) and a second line section (332), the first line section (330) comprising the first inspiratory chamber (341) and the second line section (332) comprising the second inspiratory chamber (343).

3. Interface according to Claim 2, **characterized in that** the first line section (330) comprises a first gas intake valve (331) and the second line section (332) comprises a second gas intake valve (333).

4. Interface according to Claim 1, **characterized in that** the expiratory line (431) branches into a first collection channel (3513) and a second collection channel (3533), the first collection channel (3513) comprising the first expiratory chamber (351) and the second collection channel (3533) comprising the second expiratory chamber (353).

5. Interface according to any one of the preceding claims, **characterized in that**:
- the first inspiratory chamber (341) comprises a first inspiratory sensor (3414), and the second inspiratory chamber (343) comprises a second inspiratory sensor (3434), and
- the first expiratory chamber (351) comprises a first expiratory sensor (3514), and the second expiratory chamber (353) comprises a second expiratory sensor (3534).

6. Interface according to any one of the preceding claims, **characterized in that** it comprises control means (321).

7. Interface according to Claims 3 and 6, **characterized in that** the control means (321) control at least the first and second gas intake valves (331, 333).

8. Interface according to Claims 5 and 6, **characterized in that** the first and second inspiratory and expiratory sensors (3414, 3434; 3514, 3534) are electrically connected to the control means (321).

9. Interface according to Claim 1, **characterized in that** the first and/or second inspiratory reservoir (3411, 3431) and/or the first and/or second expiratory reservoir (3511, 3531) comprise bellows, preferably circular bellows.

10. Interface according to Claim 6, **characterized in that** the control means (321) comprise at least one electronic control card and/or at least one microprocessor-based control unit.

11. Interface according to Claim 1, **characterized in that** the first and/or second inspiratory reservoir (3411, 3431) and/or the first and/or second expiratory reservoir (3511, 3531) are formed of at least one flexible material, preferably medical-grade silicone.

12. Assisted ventilation system (5, 10, 30) for a patient, comprising:
- a ventilation interface (30) according to any one of the preceding claims,
- a medical ventilator (10) fluidically connected to the first gas inlet port (32) of the main gas supply line (501) via a first patient circuit (20), and
- an additional gas source (5) fluidically connected to the second gas inlet port (33) of the additional gas supply line (500) via a connecting hose (52).

13. Assisted ventilation system according to Claim 12, **characterized in that** the additional gas source (5) is a pressurized gas cylinder containing an argon-based gas or gas mixture, in particular an argon/O₂ mixture, or a xenon-based mixture, in particular a xenon/nitrogen/O₂ mixture.
